# EUROPÄISCHE PATENTANMELDUNG

(11) **EP 4 332 207 A1**
(43) Veröffentlichungstag der Anmeldung: **06.03.2024**
(21) Anmeldenummer: 23190311.3
(22) Anmeldetag: 08.08.2023
(51) Int. Cl.: C11D 17/00, C11D 1/825, C11D 3/04, C11D 3/00, A61L 9/012, A61L 9/04

(54) **WASSERVERLUST-STABILE GELZUSAMMENSETZUNG**

(30) Priorität: 29.08.2022 DE 102022121761
(71) Anmelder: Henkel AG & Co. KGaA, 40589 Düsseldorf (DE)
(72) Erfinder: Schmeling, Marianne, 41352 Korschenbroich (DE); Poethkow, Daniela, 47809 Krefeld (DE)

(57) **Zusammenfassung**

Die Erfindung betrifft Reinigungs- und/oder Lufterfrischungsmittel enthaltend mindestens ein C16-C18 Fettalkoholalkoxylat mit einem Alkoxylierungsgrad von 15-30 EO, mindestens ein Polyglucosid und mindestens ein Salz. Ferner betrifft die Erfindung die Verwendung eines solchen Reinigungs- und/oder Lufterfrischungsmittels zum Reinigen von harten Oberflächen und/oder zum Erfrischen der Luft, sowie die Verwendung einer Kombination aus mindestens einem C16-C18 Fettalkoholalkoxylat mit einem Alkoxylierungsgrad von 15-30 EO, mindestens einem Polyglucosid und mindestens einem Salz zur Stabilisierung eines gelförmigen Reinigungs- und/oder Lufterfrischungsmittels.

## Beschreibung

Die Erfindung betrifft Reinigungs- und/oder Lufterfrischungsmittel enthaltend mindestens ein C16-C18 Fettalkoholalkoxylat mit einem Alkoxylierungsgrad von 15-30 EO, mindestens ein Polyglucosid und mindestens ein Salz. Ferner betrifft die Erfindung die Verwendung eines solchen Reinigungs- und/oder Lufterfrischungsmittels zum Reinigen von harten Oberflächen und/oder zum Erfrischen der Luft, sowie die Verwendung einer Kombination aus mindestens einem C16-C18 Fettalkoholalkoxylat mit einem Alkoxylierungsgrad von 15-30 EO, mindestens einem Polyglucosid und mindestens einem Salz zur Stabilisierung eines gelförmigen Reinigungs- und/oder Lufterfrischungsmittels.

Aus dem Stand der Technik sind Reinigungsmittel und Lufterfrischer, beispielsweise allgemein sanitäre Mittel, in Gelform bekannt. Dabei kann es sich einerseits um selbsthaftende Mittel, wie aus den Druckschriften EP 1 086 199 B1, DE 10 2004 056554 A1, EP 1 894 989 A1 und WO 2014/033259 A1 bekannt, andererseits um nicht-selbsthaftende Mittel, wie in den Druckschriften DE 197 15 872 A1 und EP 1 029 911 A1 beschrieben, handeln.

Die Vorteile von gelförmigen gegenüber festförmigen oder flüssigen Mitteln bestehen vor Allem in der leichten Dosier- und allgemein Handhabbarkeit verbunden mit einer ansprechenden Optik. Gegenüber festförmigen Mitteln besteht ein weiterer Vorteil darin, dass entsprechende Mittel generell einfacher löslich sind und entsprechend ihre Wirkung schneller entfalten können. Gegenüber flüssigen Mitteln besteht hingegen der Vorteil, dass entsprechende Mittel allgemein weniger leicht wegspülbar sind und sich auch weniger schnell verflüchtigen, beispielsweise haften gelförmige Mittel länger an Anschmutzungen als flüssige Mittel und können so ihre Wirkung über einen vergleichsweise längeren Zeitraum entfalten.

Wenn sie allerdings nicht ein einem geschlossenen System, wie beispielsweise einem Aufbewahrungsbehältnis, vorliegen und in Kontakt mit Luft sind, verlieren gelförmige Mittel allerdings schnell an Stabilität aufgrund von Wasserverlust. Auch bei erhöhten Temperaturen (> 25 °C) sind gelförmige Mittel in der Regel instabil; das Gel geht von der kubischen in die flüssige Phase über. Derart kritische Bedingungen liegen insbesondere bei der Anwendung entsprechender Mittel vor.

Diesen Nachteilen wird üblicherweise insofern begegnet, als dass der Gehalt an Tensiden und/oder Parfümen in entsprechenden Mitteln verringert wird, um eine Stabilitätserhöhung herbeizuführen. Einerseits ist die auf diese Weise erreichte Stabilitätserhöhung nichtsdestotrotz unzureichend, andererseits verschlechtert sich gleichzeitig die anwendungstechnischen Qualitäten des Mittels, beispielsweise in Bezug auf Reinigungsleistung, Schaumbildung, und Duft-/Frischeeindruck.

Es besteht folglich weiterhin Bedarf an stabilen gelförmigen Reinigungsmitteln und Lufterfrischern, welche die vorgenannten Nachteile nicht oder in reduziertem Ausmaß aufweisen.

Diese Aufgabe wird durch die hierin beschriebenen gelförmigen Reinigungs- und/oder Lufterfrischungsmittel gelöst.

In einem ersten Aspekt betrifft die vorliegende Erfindung entsprechend ein gelförmiges Reinigungs- und/oder Lufterfrischungsmittel, wobei das Mittel mindestens ein C16-C18 Fettalkoholalkoxylat mit einem Alkoxylierungsgrad von 15-30 EO, mindestens ein Polyglucosid und mindestens ein Salz umfasst.

In einem weiteren Aspekt betrifft die Erfindung auch die Verwendung einer Kombination aus mindestens einem C16-C18 Fettalkoholalkoxylat mit einem Alkoxylierungsgrad von 15-30 EO, mindestens einem Polyglucosid und mindestens einem Salz zur Stabilisierung eines gelförmigen Reinigungs- und/oder Lufterfrischungsmittels.

Schließlich betrifft die Erfindung auch die Verwendung eines erfindungsgemäßen gelförmigen Reinigungs- und/oder Lufterfrischungsmittels zum Reinigen von harten Oberflächen, insbesondere zur Reinigung von Sanitäroberflächen, und/oder zum Erfrischen der Luft, insbesondere zum Erfrischen der Luft im Sanitärbereich.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

Der Ausdrücke "ungefähr" "ca." oder "etwa", in Zusammenhang mit einem Zahlenwert, bezieht sich auf eine Varianz von ±10 % bezogen auf den angegebenen Zahlenwert, bevorzugt ±5 %, besonders bevorzugt ±1 %, noch stärker bevorzugt unter ± 0,1 %.

"Mindestens ein", wie hierin verwendet, schließt ein, ist aber nicht begrenzt auf 1, 2, 3, 4, 5, 6 und mehr. Bezogen auf einen Inhaltsstoff bezieht sich die Angabe auf die Art des Inhaltsstoffs und nicht auf die absolute Zahl der Moleküle. "Mindestens ein Tensid" bedeutet somit beispielsweise mindestens eine Art von Tensid, d.h. dass eine Art von Tensid oder eine Mischung mehrerer verschiedener Tenside gemeint sein kann. Zusammen mit Gewichtsangaben bezieht sich die Angabe auf alle Verbindungen der angegebenen Art, die in dem Produkt enthalten sind, d.h. dass das Produkt über die angegebene Menge der entsprechenden Verbindungen hinaus keine weiteren Verbindungen dieser Art enthält.

Wenn hierin auf Molmassen Bezug genommen wird, beziehen sich diese Angaben immer auf die zahlenmittlere Molmasse _{Mn}, sofern nicht explizit anders angeben. Das Zahlenmittel der Molmasse kann beispielsweise mittels Gel-Permeations-Chromatographie (GPC) gemäß DIN 55672-1:2007-08 mit THF als Eluent bestimmt werden. Die massenmittlere Molmasse M_{w} kann ebenfalls mittels GPC bestimmt werden, wie für Mₙ beschrieben.

Wann immer im Folgenden Erdalkalimetalle als Gegenionen für einwertige Anionen genannt sind, so bedeutet das, dass das Erdalkalimetall natürlich nur in der halben - zum Ladungsausgleich ausreichenden - Stoffmenge wie das Anion vorliegt.

Im Zusammenhang mit der vorliegenden Erfindung wird unter "gelförmig" eine salbenartige, pastöse und/oder eine cremeartige Konsistenz verstanden, bevorzugt ein disperses System, das aus mindestens zwei Komponenten besteht. Die feste Komponente bildet dabei ein schwammartiges, dreidimensionales Netzwerk, dessen Poren zumindest teilweise durch ein Lösungsmittel ausgefüllt sind. Aufgrund einer derart besonderen Mikro- beziehungsweise Nanostruktur ergibt sich eine insbesondere im Falle von Toilettenanwendungen besondere optische Erscheinung aufgrund der Transparenz des Gels. Darüber hinaus wird auch eine gleichmäßige Freisetzung von Additiven wie Farbstoffen und insbesondere Duftstoffzusammensetzungen ermöglicht.

"Fest", wie hierin verwendet, bezeichnet Zusammensetzungen, die bei Raumtemperatur (20 °C) und bei Standarddruck (1 bar) fest sind, also nicht flüssig, gelförmig oder gasförmig.

Bei den hierin beschriebenen Mitteln kann es sich sowohl um selbsthaftende als auch nicht-selbsthaftende Mittel handeln. "Selbsthaftend" bedeutet im Kontext der vorliegenden Erfindung, dass das Mittel geeignet ist, eigenständig, insbesondere ohne weitere Hilfsmittel, an Oberflächen zu haften. In beiden Ausgestaltungsformen kann, in verschiedenen Ausführungsformen, ein entsprechendes erfindungsgemäßes Mittel in (Kunststoff-)Behältnisse, insbesondere Mehrkammerbehältnisse, gefüllt werden, um insbesondere in der Toilette angebracht zu werden. Bei selbsthaftenden Mitteln ist auch das Befüllen eines Applikators, um das Produkt innen auf die Toilettenschüssel aufzustempeln, denkbar.

Die Begriffe "WC-Produkt", "WC-Artikel" oder "Toilettenzusatzartikel" sind im Kontext dieser Erfindung synonym zu verwenden. Bevorzugt ist das WC-Produkt ein WC-Reiniger oder ein WC-Erfrischer, z.B. ein WC-Lufterfrischer oder WC-Duftspüler. Insbesondere sind von der vorliegenden Erfindung generell sowohl selbsthaftende als auch nicht-selbsthaftende WC-Produkte erfasst. Entsprechende Produkte fallen unter den im Kontext der vorliegenden Erfindung ebenfalls verwendeten Begriff "sanitäre Mittel".

Im Zusammenhang mit der vorliegenden Erfindung wird der Ausdruck "sanitäres Mittel" im Sinne einer "Zusammensetzung, die geeignet ist zur Verwendung als sanitäres Mittel", verwendet.

Im Zusammenhang mit der vorliegenden Erfindung wird unter "Sanitärgegenstand" bevorzugt ein Gegenstand verstanden, der üblicherweise im Bereich von privaten oder öffentlichen Bädern und Toiletten Verwendung findet. Insbesondere sind unter Sanitärgegenstand Toiletten, bevorzugt Toilettenschüsseln, Urinale, Pissoirs, aber auch Handspülbecken zu verstehen.

Im Zusammenhang mit der vorliegenden Erfindung steht der Ausdruck "EO" für Alkoxygruppen im Allgemeinen. So bedeutet beispielsweise der Ausdruck "20 EO", die Anwesenheit von 20 Alkoxygruppen, die besonders bevorzugt als Block im betreffenden Molekül vorliegen und noch weiter bevorzugt als Endgruppen im betreffenden Molekül angeordnet sind. Insbesondere steht der Ausdruck "EO" für die Gruppe enthaltend Ethoxy-, Propoxy- und Butoxygruppen, weiter bevorzugt für die Gruppe enthaltend Ethoxy- und Propoxygruppen und besonders bevorzugt nur für Ethoxygruppen.

Die Erfindung betrifft gelförmige Reinigungs- und/oder Lufterfrischungsmittel, wobei das Mittel mindestens ein C16-C18 Fettalkoholalkoxylat mit einem Alkoxylierungsgrad von 15-30 EO, mindestens ein Polyglucosid und mindestens ein Salz umfasst.

"Fettalkoholalkoxylate" im Sinne der vorliegenden Erfindung sind Verbindungen, die wenigstens einen hydrophoben Rest und wenigstens einen hydrophilen Rest umfassen, wobei der hydrophile Rest 15 bis 30 EO, beispielsweise 15, 16, 17, 18, 19, 20, 21, 22, 23, 24,2 5, 26, 27, 28, 29, oder 30 EO, aufweist, die an den hydrophoben Rest gebunden ist. Der hydrophobe Rest leitet sich insbesondere von aliphatischen, langkettigen, einwertigen Alkoholen ab. Der hydrophobe Rest ist bevorzugt verzweigt. Der hydrophobe Rest weist zwischen 16 bis 18 Kohlenstoffatome auf und kann auch ein- oder mehrfach ungesättigt sein, wobei vollständig gesättigte Reste bevorzugt sind.

Als hydrophile Reste können Polyalkoxygruppen, vorzugsweise Polyethoxy-, Polypropoxy- oder Polybutoxy- oder auch gemischte Polyalkoxygruppen wie beispielsweise Poly(ethoxypropoxy)gruppen eingesetzt werden. Ein Fettalkoholalkoxylat, welches sowohl Ethoxy als auch Propoxygruppen aufweist, wird im Zusammenhang mit der Erfindung als ein gemischtes Fettalkoholethoxypropoxylat bezeichnet.

In verschiedenen Ausführungsformen ist das mindestens eine Fettalkoholalkoxylat ausgewählt aus der Gruppe bestehend aus Fettalkoholethoxylaten, Fettalkoholpropoxylaten, Fettalkoholbutoxyalten und gemischten Fettalkoholalkoxylaten.

Besonders bevorzugt als hydrophile Gruppe ist ein Polyethoxyrest.

Als hydrophober Rest sind bevorzugt lineare, d.h. unverzweigte Alkylreste geeignet. Hierdurch wird die Eignung zur Netzwerkbildung begünstigt. Geradzahlige Alkylreste sind bevorzugt - wegen deren besseren biologischen Abbaubarkeit. Wie bereits erwähnt besteht der Alkylrest aus 16 bis 18 Kohlenstoffatomen.

In verschiedenen Ausführungsformen ist das mindestens eine Fettalkoholalkoxylat, wie voranstehend definiert, in einer Menge von ungefähr 15 bis 45 Gew.-%, beispielsweise in einer Menge von 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, oder 45 Gew.-%, vorzugsweise in einer Menge von ungefähr 20 bis 40 Gew.-%, jeweils bezogen auf das Gesamtgewicht des Mittels, in diesem enthalten.

Darüber hinaus kann das erfindungsgemäß Mittel, zusätzlich zu dem mindestens einen Fettalkoholalkoxylat, weitere nicht-ionische Tenside, beispielsweise weitere Fettalkoholalkoxylate, die sich von dem vorstehend definierten mindestens einem Fettalkoholalkoxylat unterscheiden, enthalten.

In verschiedenen Ausführungsformen ist vorgesehen, dass das Mittel weniger als 15 Gew.-%, vorzugsweise weniger als 10 Gew.-%, noch bevorzugter weniger als 5 Gew.-%, noch bevorzugter weniger als 3 Gew.-%, jeweils bezogen auf das Gesamtgewicht des Mittels, an Fettalkoholalkoxylaten mit einem Alkoxylierungsgrad von ≤ 8 EO umfasst. Auf diese Weise kann die Stabilität des Mittels weiter verbessert werden.

Polyglucoside sind im Stand der Technik grundsätzlich bekannt. So schließen im Rahmen der vorliegenden Erfindung geeignete Polyglucoside Alkylpolyglykoside, Glyceringlykolipide und Saccharoseester ein.

Im Rahmen der erfindungsgemäßen Lehre bevorzugte Polyglucoside sind die Alkylpolyglykoside sowie deren Derivate, insbesondere ihre Ether und Ester. Bei den Ethern handelt es sich um die Produkte der Reaktion einer oder mehrerer, vorzugsweise einer Zuckerhydroxygruppe mit einer eine oder mehrere Hydroxygruppen enthaltenden Verbindung, beispielsweise C₁₋₂₂-Alkoholen oder Glykolen wie Ethylen- und/oder Propylenglykol, wobei die Zuckerhydroxygruppe auch Polyethylenglykol- und/oder Polypropylenglykolreste tragen kann. Die Ester sind die Reaktionsprodukte einer oder mehrerer, vorzugsweise einer, Zuckerhydroxygruppe mit einer Carbonsäure.

Die Alkylpolyglykoside (APG) sind im Rahmen der erfindungsgemäßen Lehre besonders bevorzugte Polyglucoside und genügen vorzugsweise der allgemeinen Formel R'O(AO)₃[G]ₓ, in der R' für einen linearen oder verzweigten, gesättigten oder ungesättigten Alkylrest mit 6 bis 22, vorzugsweise 6 bis 18, insbesondere 8 bis 16, besonders bevorzugt 8 bis 14 Kohlenstoffatomen, [G] für einen glykosidisch verknüpften Zuckerrest und x für eine Zahl von 1 bis 10 sowie AO für eine Alkylenoxygruppe, z.B. eine Ethylenoxy- oder Propylenoxygruppe, und a für den mittleren Alkoxylierungsgrad von 0 bis 20 stehen. Hierbei kann die Gruppe (AO)₃ auch verschiedene Alkylenoxyeinheiten enthalten, z.B. Ethylenoxy- oder Propylenoxyeinheiten, wobei es sich dann bei a um den mittleren Gesamtalkoxylierungsgrad, d.h. die Summe aus Ethoxylierungs- und Propoxylierungsgrad, handelt. Soweit nachfolgend nicht näher bzw. anders ausgeführt, handelt es sich bei den Alkylresten R' der APG um lineare ungesättigte Reste mit der angegebenen Zahl an Kohlenstoffatomen.

APG sind nichtionische Tenside und stellen bekannte Stoffe dar, die nach den einschlägigen Verfahren der präparativen organischen Chemie erhalten werden können. Die Indexzahl x gibt den Oligomerisierungsgrad (DP-Grad) an, d h die Verteilung von Mono- und Oligoglykosiden, und steht für eine Zahl zwischen 1 und 10. Während x in einer gegebenen Verbindung stets ganzzahlig sein muss und hier vor allem die Werte x = 1 bis 6 annehmen kann, ist der Wert x für ein bestimmtes Alkylglykosid eine analytisch ermittelte rechnerische Größe, die meistens eine gebrochene Zahl darstellt. Vorzugsweise werden Alkylglykoside mit einem mittleren Oligomerisierungsgrad x von 1,1 bis 3,0 eingesetzt. Aus anwendungstechnischer Sicht sind solche Alkylglykoside bevorzugt, deren Oligomensierungsgrad kleiner als 1,7 ist und insbesondere zwischen 1,2 und 1,6 liegt. Als glykosidischer Zucker wird vorzugsweise Xylose, insbesondere aber Glucose verwendet. Der Alkyl- bzw. Alkenylrest R' kann sich von primären Alkoholen mit 8 bis 18, vorzugsweise 8 bis 14 Kohlenstoffatomen ableiten. Typische Beispiele sind Capronalkohol, Caprylalkohol, Caprinalkohol und Undecylalkohol sowie deren technische Gemische, wie sie beispielsweise im Verlauf der Hydrierung von technischen Fettsäuremethylestern oder im Verlauf der Hydrierung von Aldehyden aus der ROELENschen Oxosynthese anfallen.

Vorzugsweise leitet sich der Alkyl- bzw. Alkenylrest R' aber von Laurylalkohol, Myristylalkohol, Cetylalkohol, Palmoleylalkohol, Stearylalkohol, Isostearylalkohol oder Oleylalkohol ab. Weiterhin sind Elaidylalkohol, Petroselmylalkohol, Arachidylalkohol, Gadoleylalkohol, Behenylalkohol, Erucylalkohol sowie deren technische Gemische zu nennen.

Besonders bevorzugte APG sind nicht alkoxyliert (a = 0) und genügen Formel RO[G]ₓ, in der R wie zuvor für einen linearen oder verzweigten, gesättigten oder ungesättigten Alkylrest mit 4 bis 22 Kohlenstoffatomen, [G] für einen glykosidisch verknüpften Zuckerrest, vorzugsweise Glucoserest, und x für eine Zahl von 1 bis 10, bevorzugt 1,1 bis 3, insbesondere 1,2 bis 1,6, stehen. Dementsprechend bevorzugte Alkylpolyglykoside sind beispielsweise C₈₋₁₀- und ein C₁₂₋₁₄-Alkylpolyglucosid mit einem DP-Grad von 1,4 oder 1,5, insbesondere C₈₋₁₀-Alkyl-1,5-glucosid und C₁₂₋₁₄-Alkyl-1,4-glucosid.

In verschiedenen Ausführungsformen ist das mindestens eine Polyglucosid, wie voranstehend definiert, in einer Menge von ungefähr 1 bis 20 Gew.-%, beispielsweise in einer Menge von 1, 2, 3, 4, 5, 6, 7, 8, 9, 10, 11, 12, 13, 14, 15, 16, 17, 18, 19, oder 20 Gew.-%, vorzugsweise in einer Menge von ungefähr 2 bis 17 Gew.-%, noch bevorzugter in einer Menge von ungefähr 2 bis 15 Gew.-%, jeweils bezogen auf das Gesamtgewicht des Mittels, in diesem enthalten.

Das erfindungsgemäße Mittel enthält weiterhin mindestens ein Salz. Dabei kann es sich um anorganische und/oder organische Salze handeln. Bevorzugt sind in diesem Zusammenhang anorganische Salze.

Erfindungsgemäß einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben. Insbesondere bevorzugt ist Natriumchlorid.

In bevorzugten Ausführungsformen sind die erfindungsgemäßen Mittel phosphat- und/oder phosphonatfrei.

Bei den erfindungsgemäß einsetzbaren organischen Salzen handelt es sich insbesondere um farblose wasserlösliche Alkalimetall-, Erdalkalimetall-, Ammonium-, Aluminium- und/oder Übergangsmetallsalze der Carbonsäuren, einschließlich der Dicarbonsäuren. Vorzugsweise sind die Salze ausgewählt aus der Gruppe umfassend Formiat, Acetat, Propionat, Citrat, Malat, Maleat, Tartrat, Succinat, Malonat, Oxalat, Lactat, Fumarat, Adipat, Succinat, Glutarat, Methylglycindiessigsäuretrinatriumsalz sowie Gemische derselben.

In verschiedenen Ausführungsformen ist das mindestens eine Salz, wie voranstehend beschrieben, in einer Menge von ungefähr 0,1 bis 10 Gew.-%, beispielsweise in einer Menge von 0,1, 0,2, 0,3, 0,4, 0,5, 0,6, 0,7, 0,8, 0,9, 1,1, 1,2, 1,3, 1,4, 1,5, 1,6, 1,7, 1,8, 1,9, 2,0, 2,5, 3,0, 3,5, 4,5, 5,5, 6,0, 6,5, 7,0, 7,5, 8,0, 8,5, 9,0, 9,5, oder 10,0 Gew.-%, vorzugsweise in einer Menge von ungefähr 1 bis 8 Gew.-%, jeweils bezogen auf das Gesamtgewicht des Mittels, in diesem enthalten.

In verschiedenen Ausführungsformen umfasst das Mittel des Weiteren mindestens ein Polyol, vorzugsweise in einer Menge von ungefähr 3 Gew.-% bis 35 Gew.-%, beispielsweise in einer Menge von 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, oder 35 Gew.-%, bevorzugter in einer Menge von ungefähr 2 bis 15 Gew.-%, insbesondere in einer Menge von ungefähr 5 bis 15 Gew.-%, jeweils bezogen auf das Gesamtgewicht des Mittels, wobei es sich bei dem mindestens einen Polyol vorzugsweise um Glycerin handelt.

Vorteilhaft enthält ein erfindungsgemäßes Mittel, zusätzlich zu den voranstehend beschriebenen Komponenten, des Weiteren mindestens einen weiteren Inhaltsstoff, beispielsweise 1, 2, 3, 4, 5, 6, 7, 8, 9, oder 10 weitere Inhaltsstoffe, vorzugsweise mindestens zwei weitere Inhaltsstoffe, ausgewählt aus der Gruppe bestehend aus weiteren Tensiden, Bleichmitteln, Lösungsmitteln, Verdickungsmitteln, Duft- und/oder Riechstoffen, Wirkstoffen zur Verringerung von Schlechtgerüchen, Parfümboostern, Farbstoffen, Konservierungsstoffen, antimikrobiellen Wirkstoffen, pH-Stellmitteln, Korrosionsinhibitoren, Enzymen, Mikroorganismen, Wirkstoffen zur Biofilmentfernung, Wirkstoffen zur Inhibierung der Kalkablagerung, Wirkstoffen zur Verminderung der Schmutzhaftung und Wirkstoffen zur Verbesserung der Verarbeitbarkeit.

In verschiedenen Ausführungsformen enthält das Mittel mindestens ein anionisches Tensid.

In verschiedenen Ausführungsformen ist allerdings vorgesehen, dass das Mittel anionische Tenside in einer Menge von weniger als 20 Gew.-%, vorzugsweise weniger als 15 Gew.-%, noch bevorzugter weniger als 10 Gew.-%, insbesondere weniger als 5 Gew.-%, jeweils bezogen auf das Gesamtgewicht des Mittels, aufweist. Auf diese Weise kann die Stabilität des Mittels weiter verbessert werden.

In verschiedenen Ausführungsformen ist vorgesehen, dass das Mittel im Wesentlichen frei ist von Natriumlaurylethersulfaten, bevorzugt von Laurylethersulfaten, noch bevorzugter von Fettsäureethylethersulfaten. Auf diese Weise kann die Stabilität des Mittels weiter verbessert werden.

Bevorzugt handelt es sich bei im Kontext der vorliegenden Erfindung eingesetzten Lösungsmitteln um polare Lösungsmittel, weiter bevorzugt wässrige Lösungsmittel, und insbesondere um Wasser. In verschiedenen Ausführungsformen ist bevorzugt, dass die Gelzusammensetzung Wasser in einer Menge zwischen 25 und 70 Gew.-%, bezogen auf die Gesamtmenge der Gelzusammensetzung, enthält.

Der Einsatz von Duft- und/oder Riechstoffen ermöglicht eine Verbesserung der Raumluft im Zuge der Anwendung des Mittels, trägt aber darüber hinaus auch zu einem angenehmen Gesamtprodukteindruck beim Endverbraucher bei, beispielsweise bereits beim Öffnen der Verpackung des Mittels.

Im Kontext der erfindungsgemäßen Mittel geeignete Konservierungsstoffe sind beispielsweise unter den Handelsnamen Acticide B 20, Acticide MBR 1 und Acticide SR 1500 erhältlich. Bevorzugt umfasst das als Konservierungsstoffe eines oder mehrere aus der Gruppe enthaltend Isothiazolinon, Phenoxyethanol, Methylisothiazolinon und Benzisothiazolinone (BIT). Bevorzugt und abhängig von der Art des Konservierungsstoffes ist dieser in Mengen von 0,0001 bis 1 Gew.-%, bezogen auf das Gesamtgewicht des Mittels, in diesem enthalten.

In verschiedenen Ausführungsformen kann das Mittel einen pH-Wert von 1 bis 10, bevorzugt von 3 bis 8, aufweisen, gemessen in einer 10-% Lösung des Mittels in Wasser. Dies ist insofern vorteilhaft, als dass auf diese Weise die Ausbildung eines Biofilms eingeschränkt oder sogar weitgehend verhindert werden kann.

In verschiedenen Ausführungsformen ist das hierin beschriebene gelförmige Mittel stabil bis zu einer Temperatur von ungefähr 50 °C, bevorzugt ungefähr 40 °C, noch bevorzugter ungefähr 35 °C, noch bevorzugter ungefähr 30 °C, am meisten bevorzugt ungefähr 25 °C gelförmig.

Gemäß einer bevorzugten Ausführungsform weist das Gel eine kubische Phase auf.

Die Kombination aus mindestens einem C16-C18 Fettalkoholalkoxylat mit einem Alkoxylierungsgrad von 15-30 EO, wie voranstehend beschrieben, mindestens einem Polyglucosid, wie voranstehend beschrieben, und mindestens einem Salz, wie voranstehend beschrieben, ermöglicht die Stabilisierung eines entsprechenden gelförmigen Reinigungs- und/oder Lufterfrischungsmittels, insbesondere gegen Wasserverlust.

Entsprechend betrifft die vorliegende Erfindung in einem weiteren Aspekt die Verwendung einer Kombination aus mindestens einem C16-C18 Fettalkoholalkoxylat mit einem Alkoxylierungsgrad von 15-30 EO, mindestens einem Polyglucosid und mindestens einem Salz zur Stabilisierung eines gelförmigen Reinigungs- und/oder Lufterfrischungsmittels. Sämtliche Definitionen und Ausführungsformen betreffend das mindestens eine Fettalkoholalkoxylat, das mindestens eine Polyglucosid und das mindestens eine Salz sind analog auch auf die erfindungsgenmäße Verwendung der entsprechenden Kombination anzuwenden.

Ein weiterer Aspekt der Erfindung betrifft die Verwendung des erfindungsgemäßen gelförmigen Reinigungs- und/oder Lufterfrischungsmittels. Insbesondere betrifft die vorliegende Erfindung die Verwendung eines erfindungsgemäßen gelförmigen Reinigungs- und/oder Lufterfrischungsmittels zum Reinigen von harten Oberflächen, insbesondere zur Reinigung von Sanitäroberflächen, und/oder zum Erfrischen der Luft, insbesondere zum Erfrischen der Luft im Sanitärbereich.

Entsprechend handelt es sich bei dem erfindungsgemäßen gelförmigen Reinigungs- und/oder Lufterfrischungsmittel, gemäß einiger Ausführungsformen, insbesondere um ein sanitäres Produkt, insbesondere um ein WC-Produkt.

Das Anhaften eines erfindungsgemäßen selbsthaftenden Mittels gemäß der erfindungsgemäßen Verwendung findet bevorzugt derart statt, dass das Mittel unmittelbar auf der Oberfläche des Sanitärgegenstandes haftet. Anders ausgedrückt haftet das Mittel selbstständig auf der Oberfläche des Sanitärgegenstandes.

In einer bevorzugten Ausführungsform wird das Anhaften des Mittels mittels eines Applikators durchgeführt. Hierzu eignen sich beispielsweise Spritzen im weitesten Sinne, die das Mittel enthalten und aus denen das Mittel herausgedrückt werden kann, um dieses auf der Oberfläche des Sanitärgegenstandes zu platzieren.

Die erreichte Haftung an dem Sanitärgegenstand, bevorzugt selbst bei einer Anbringung an einer senkrechten Fläche, ist derart, dass sich das Mittel sogar unter der zusätzlichen Krafteinwirkung von Spülwasserströmen nicht auf einmal ablöst.

Das Mittel ist bevorzugt erst nach einer größeren Anzahl von Spülvorgängen abspülbar. Die Anzahl der Spülvorgänge richtet sich nach der Zusammensetzung des jeweiligen Mittels und ferner nach der aufgebrachten Menge sowie der Form des Mittels.

Das Mittel kann auch gleichzeitig an mehreren Stellen des Sanitärgegenstands angehaftet werden. Beispielsweise kann das Mittel sowohl auf der rechten als auch auf der linken Seite einer Toilettenschüssel aufgebracht werden. Dies ermöglicht eine gleichmäßigere Reinigungswirkung als die Anbringung nur auf einer Seite.

Gemäß einer weiteren Ausführungsform kann das Mittel an einem Sanitärgegenstand an verschiedenen Stellen in unterschiedlichen Zusammensetzungen angehaftet werden. Dies ermöglicht beispielsweise, dass z.B. zwei unterschiedliche Parfümierungsstoffe bei örtlicher Trennung zu einer gemeinsamen Beduftung der Toilette eingesetzt werden können.

Darüber hinaus haben die Erfinder festgestellt, dass das hier beschriebene Mittel auch vorteilhaft in einem WC Körbchen vorgesehen sein kann. Ein weiterer Aspekt betrifft ein System aufweisend das Mittel gemäß der Erfindung und ein WC Körbchen mit wenigstens einer Kammer, wobei das Mittel in der Kammer des WC Körbchens vorliegt.

In einer bevorzugten Ausführungsform ist die Kammerhülle des WC Körbchens auf wenigstens einer Seite vollständig geschlossen und die Zusammensetzung an der vollständig geschlossenen Seite der Kammerhülle angehaftet.

Eine solches Mittel in einem WC Körbchen ist ein optisch attraktives, transparentes Produkt mit einer vorteilhaften Freisetzungscharakteristik in Bezug auf Farb- und insbesondere Duftstoffe, die in der Zusammensetzung als Additive enthalten sein können.

Durch die ggf. selbsthaftenden Eigenschaften des Gels kann dieses an der Oberfläche des WC Körbchens haften. Dies hat den Vorteil, dass das im Körbchen enthaltene Gel nicht wie bei üblichen Toilettensteinen von außen nach innen aufgelöst wird, sondern ein Auflöseverhalten dahingehend aufweist, dass die Mengen an Gel, die unmittelbar an der Kammerwand des WC Körbchens haften, zuletzt abgespült werden. Hierdurch ergibt sich insbesondere ein verbessertes Freisetzungsprofil sowie ein optisch ansprechenderes Erscheinungsbild über die gesamte Lebensdauer des WC-Produktes. Da sich die von außen für den Benutzer erkennbaren Bereiche des Gels, zum Beispiel bei Verwendung eines transparenten WC Körbchens, erst ganz am Ende der Lebenszeit ablösen, wird sich das ursprüngliche Erscheinungsbild des WC-Produktes bis zum vollständigen Aufbrauchen des Gleichen kaum ändern. Dies stellt allerdings besondere Anforderungen an das Abspülverhalten des Gels von der Oberfläche der Kammer, in welcher das Gel im WC Körbchen enthalten ist.

Ein unzureichendes Abspülverhalten von der inneren Kammeroberfläche des WC Körbchens führt bei Verwendung von transparenten WC Körbchen zu unansehnlichen da ungleichmäßig verteilten Gelresten an der Oberfläche der Kammer. WC Körbchen weisen in der Regel eine stark gebogene bzw. gekrümmte innere Kammeroberfläche auf (beispielsweise bei sphärisch ausgebildeten WC Körbchen). Gegenüber den relativ glatten und ungekrümmten Oberflächen von Toilettenschüsseln, an denen sanitäre Mittel direkt ohne WC Körbchen angebracht werden können, ist bei Verwendung von WC Körbchen mit einem selbsthaftenden Gel eine ausgeprägtere Rückstandsbildung zu beobachten. Eine Verbesserung des Abspülverhalten ist somit insbesondere bei Verwendung mit einem WC Körbchen notwendig.

Bevorzugt ist die Kammerhülle als Kugel, d.h. sphärisch ausgebildet, wobei die Kammerhülle einstückig oder zweistückig aus einer ersten Halbkugel und einer zweiten Halbkugel besteht, die respektive eine erste Seite der Kammerhülle und eine zweite Seite der Kammerhülle darstellen. Die erste Seite kann als Vorderseite bezeichnet werden, d.h. die im Benutzungszustand zugängliche bzw. sichtbare und der Toilettenschüsselwand abgewandte Seite, während die zweite Seite als Rückseite bezeichnet werden kann, d.h. die im Benutzungszustand nicht unmittelbar zugängliche bzw. sichtbare und der Toilettenschüsselwand und dem daran entlanglaufenden Spülwasser zugewandte Seite. Die vorstehende Einteilung der Kammerhülle in zwei Seiten kann auch auf weitere Kammerhüllengeometrien, z.B. rechteckige Kammerhüllengeometrien, entsprechend übertragen werden.

Die Vorderseite der Kammerhülle weist bevorzugt keine Öffnung auf. Dies verhindert zum einen, dass ein Kind oder ein Haustier in Kontakt mit einer in der Kammer vorhandenen Wirksubstanz geraten kann. Ferner kann dadurch, dass die erste Seite der Kammerhülle keine Öffnung aufweist, die Wirksubstanz beim Herstellungsprozess durch eine Öffnung der zweiten Seite in das Körbchen eingefüllt werden. Bei WC-Körben mit einer oder mehrerer Öffnungen auf der ersten Seite würde die Wirksubstanz bei der Befüllung nicht im Körbchen verbleiben, sondern durch dieses hindurchlaufen. Vorteilhaft kann ein WC Körbchen gemäß der beschriebenen Ausführungsform mit einer Wirksubstanz befüllt werden, wobei die Substanz sich erst nach der Befüllung verfestigt. Es ist also nicht notwendig, dass die Wirksubstanz zu dergewünschten Geometrie (z.B. einer Kugel) vorgeformt wird und erst dann in die Kammer eingebracht wird. Vielmehr wird die Formgebung dadurch erreicht, dass die Wirksubstanz in die Kammer eingefüllt wird und die Kammergeometrie bei der Verfestigung der Wirksubstanz als Matrize fungiert. Insbesondere ermöglicht die beschriebene Ausführungsform, pastöse Mittel als Wirksubstanzen einzusetzen, da solche pastösen gelförmigen Mittel aufgrund ihrer Konsistenz und "Klebrigkeit" nur schwer vorgeformt und in ihrer endgültigen Form in die Kammer des Körbchens eingebracht werden können.

Ein weiterer Aspekt der Erfindung betrifft die Verwendung des Systems umfassend die Schritte: (a) Bereitstellen des Systems aufweisend das Mittel gemäß der Erfindung und ein WC Körbchen mit wenigstens einer Kammer, wobei das Mittel in der Kammer des WC Körbchens vorliegt (b) Anbringen des Systems an einem Sanitärgegenstand.

Es versteht sich, dass alle Ausführungsformen, die hier in Bezug auf die gelförmigen Reinigungs- und/oder Lufterfrischungsmittel offenbart werden, in gleicher oder ähnlicher Weise auf die Verfahren und Verwendungen dieser Produkte anwendbar sind, und umgekehrt.

Die folgenden Beispiele dienen zur Veranschaulichung der vorliegenden Erfindung. Da diese Beispiele nur zur Veranschaulichung dienen, sollte die Erfindung nicht als darauf beschränkt angesehen werden.

### Beispiele:

Es wurden das erfindungsgemäße Mittel E1 sowie eine Vergleichsformulierung V1 bereitgestellt.

Die Mengen der jeweilig enthaltenen Rohstoffe sind in Gewichtsprozent (Gew.-%) bezogen auf die Gesamtmenge der jeweiligen Zusammensetzung in nachfolgender Tabelle 1 angegeben:

**Tabelle 1: Formulierungsrezepturen**

| | **Formulierung V1** | **Formulierung E1** |
|---|---|---|
| **Inhaltsstoffe** | [Gew.-%] | [Gew.-%] |
| Fettalkoholethoxylat, 25 EO | 25 - 35 | 25 - 35 |
| Fettalkoholethoxylat, 8 EO | 25 - 35 | |
| Alkylpolyglucosid (D-Glucopyranose, C10-C16 Alkylglycoside, oligomer | | 3 - 15 |
| Natriumlaurethsulfat | 1 - 10 | |
| Glycerin | | 5 - 15 |
| Salz | | 1 - 7 |
| Parfum | 1 - 5 | 1 - 5 |
| Farbstoff | + | + |
| Konservierungsmittel | + | + |
| Wasser | ad 100 | ad 100 |

Die Evaluierung der Stabilität der beiden Formulierungen E1 und V1 erfolgte anhand visueller Abmusterung der jeweils über mehrere Tage bei 30 °C und einer relativen Luftfeuchtigkeit von 50 % gelagerten Produkte. Die Ergebnisse dieser Evaluierung sind in nachfolgender Tabelle 2 angegeben. Dabei bedeutet die Beurteilung "1", dass keine Änderung im Produkt aufgetreten ist, die Beurteilung "2", dass das Produkt leicht geschmolzen ist, die Beurteilung "3", dass das Produkt vollständig geschmolzen ist.

**Tabelle 2: Produktstabilität bei 30 °C/ 50 % relativer Feuchte**

| | **V1** | **E1** |
|---|---|---|
| Tag 1 | 1 | 1 |
| Tag 2 | 2 | 1 |
| Tag 3 | 2 | 1 |
| Tag 4 | 3 | 1 |

## Patentansprüche

1. Gelförmiges Reinigungs- und/oder Lufterfrischungsmittel, wobei das Mittel mindestens ein C16-C18 Fettalkoholalkoxylat mit einem Alkoxylierungsgrad von 15-30 EO, mindestens ein Polyglucosid und mindestens ein Salz umfasst.

2. Das gelförmige Reinigungs- und/oder Lufterfrischungsmittel gemäß Anspruch 1, wobei das mindestens eine Fettalkoholalkoxylat in einer Menge von ungefähr 15 bis 45 Gew.-%, vorzugsweise in einer Menge von ungefähr 20 bis 40 Gew.-%, jeweils bezogen auf das Gesamtgewicht des Mittels, enthalten ist.

3. Das gelförmige Reinigungs- und/oder Lufterfrischungsmittel gemäß Anspruch 1 oder Anspruch 2, wobei das mindestens eine Fettalkoholalkoxylat ausgewählt ist aus der Gruppe bestehend aus Fettalkoholethoxylaten, Fettalkoholpropoxylaten, Fettalkoholbutoxyalten und gemischten Fettalkoholalkoxylaten.

4. Das gelförmige Reinigungs- und/oder Lufterfrischungsmittel gemäß einem der Ansprüche 1 bis 3, wobei das mindestens eine Fettalkoholalkoxylat ausgewählt ist aus der Gruppe bestehend aus Fettalkoholethoxylaten.

5. Das gelförmige Reinigungs- und/oder Lufterfrischungsmittel gemäß einem der Ansprüche 1 bis 4, wobei das mindestens eine Polyglucosid in einer Menge von ungefähr 1 bis 20 Gew.-%, vorzugsweise in einer Menge von ungefähr 2 bis 15 Gew.-%, jeweils bezogen auf das Gesamtgewicht des Mittels, enthalten ist.

6. Das gelförmige Reinigungs- und/oder Lufterfrischungsmittel gemäß einem der Ansprüche 1 bis 5, wobei das mindestens eine Salz in einer Menge von ungefähr 0,1 bis 10 Gew.-%, vorzugsweise in einer Menge von ungefähr 1 bis 8 Gew.-%, jeweils bezogen auf das Gesamtgewicht des Mittels, enthalten ist.

7. Das gelförmige Reinigungs- und/oder Lufterfrischungsmittel gemäß einem der Ansprüche 1 bis 7, wobei das Mittel des Weiteren mindestens ein Polyol umfasst, vorzugsweise in einer Menge von ungefähr 3 Gew.-% bis 35 Gew.-%, insbesondere 5 bis 15 Gew.-%, jeweils bezogen auf das Gesamtgewicht des Mittels, wobei es sich bei dem mindestens einen Polyol vorzugsweise um Glycerin handelt.

8. Das gelförmige Reinigungs- und/oder Lufterfrischungsmittel gemäß einem der Ansprüche 1 bis 7, wobei das Mittel weniger als 15 Gew.-%, vorzugsweise weniger als 10 Gew.-%, noch bevorzugter weniger als 5 Gew.-%, noch bevorzugter weniger als 3 Gew.-%, jeweils bezogen auf das Gesamtgewicht des Mittels, an Fettalkoholalkoxylaten mit einem Alkoxylierungsgrad von ≤ 8 EO umfasst.

9. Das gelförmige Reinigungs- und/oder Lufterfrischungsmittel gemäß einem der Ansprüche 1 bis 8, wobei das Mittel anionische Tenside in einer Menge von weniger als 20 Gew.-%, vorzugsweise weniger als 15 Gew.-%, noch bevorzugter weniger als 10 Gew.-%, insbesondere weniger als 5 Gew.-%, jeweils bezogen auf das Gesamtgewicht des Mittels, aufweist.

10. Das gelförmige Reinigungs- und/oder Lufterfrischungsmittel gemäß einem der Ansprüche 1 bis 9, wobei das Mittel im Wesentlichen frei ist von Natriumlaurylethersulfaten, bevorzugt von Laurylethersulfaten, noch bevorzugter von Fettsäureethylethersulfaten.

11. Das gelförmige Reinigungs- und/oder Lufterfrischungsmittel gemäß einem der Ansprüche 1 bis 10, wobei das Mittel einen pH-Wert von 1 bis 10, bevorzugt von 3 bis 8, aufweist, gemessen in einer 10-% Lösung des Mittels in Wasser.

12. Das gelförmige Reinigungs- und/oder Lufterfrischungsmittel gemäß einem der Ansprüche 1 bis 11, des Weiteren umfassend mindestens einen weiteren Inhaltsstoff, vorzugsweise mindestens zwei weitere Inhaltsstoffe ausgewählt aus der Gruppe bestehend aus weiteren Tensiden, Bleichmitteln, Lösungsmitteln, Verdickungsmitteln, Duft- und/oder Riechstoffen, Wirkstoffen zur Verringerung von Schlechtgerüchen, Parfümboostern, Farbstoffen, Konservierungsstoffen, antimikrobiellen Wirkstoffen, pH-Stellmitteln, Korrosionsinhibitoren, Enzymen, Mikroorganismen, Wirkstoffen zur Biofilmentfernung, Wirkstoffen zur Inhibierung der Kalkablagerung, Wirkstoffen zur Verminderung der Schmutzhaftung und Wirkstoffen zur Verbesserung der Verarbeitbarkeit.

13. Verwendung einer Kombination aus mindestens einem C16-C18 Fettalkoholalkoxylat mit einem Alkoxylierungsgrad von 15-30 EO, mindestens einem Polyglucosid und mindestens einem Salz zur Stabilisierung eines gelförmigen Reinigungs- und/oder Lufterfrischungsmittels.

14. Verwendung eines gelförmigen Reinigungs- und/oder Lufterfrischungsmittels gemäß einem der Ansprüche 1 bis 12 zum Reinigen von harten Oberflächen, insbesondere zur Reinigung von Sanitäroberflächen, und/oder zum Erfrischen der Luft, insbesondere zum Erfrischen der Luft im Sanitärbereich.
